# EUROPEAN PATENT APPLICATION

(11) **EP 4 119 130 A1**
(43) Date of publication of application: **18.01.2023**
(21) Application number: 21769015.5
(22) Date of filing: 09.03.2021
(51) Int. Cl.: A61K 9/16, A61K 47/10, A61K 47/12, A61K 47/14, A61K 47/32, A61K 47/38

(54) **GRANULES AND PREPARATION USING SAME**

(30) Priority: 11.03.2020 US 202062988077 P
(71) Applicant: Sawai Pharmaceutical Co., Ltd., Yodogawa-ku Osaka-shi Osaka 532-0003 (JP)
(72) Inventor: YOSHIHARA Naoki, Osaka City, Osaka 532-0003 (JP); HASHIMOTO Shota, Osaka City, Osaka 532-0003 (JP); KIMATA Ryota, Osaka City, Osaka 532-0003 (JP)
(74) Representative: dompatent von Kreisler Selting Werner - Partnerschaft von Patent- und Rechtsanwälten mbB
(86) International application number: PCT/JP2021/009297
(87) International publication number: WO 2021/182469

(57) **Abstract**

According to one embodiment of the present invention, granules having a high content of an active ingredient and a high uniformity of particle size are provided. Alternatively, according to one embodiment of the present invention, a preparation containing granules having a high content of an active ingredient and a high uniformity of particle size is provided. According to one embodiment of the present invention, a granule is provided that comprises a nuclear material, a melt component layer arranged on a surface of the nuclear material, and an active ingredient-containing layer arranged on a surface of the melt component layer, wherein the melt component layer contains a first melt component and the active ingredient-containing layer contains an active ingredient and a second melt component or a polymer having compatibility with the first melt component.

## Description

### TECHNICAL FIELD

The present invention relates to granules containing an active ingredient at a high content and a preparation using the same.

### BACKGROUND ART

In order to improve the manufacturability of a pharmaceutical preparation, the active ingredient is granulated with various additive agents. The granulation method is divided into wet granulation and dry granulation depending on the presence or absence of a solvent. When granulating an active ingredient which is unstable in water, a dry granulation method which does not use a solvent is selected. Among the dry granulation methods, a melt granulation method in which an additive agent is melted by heat and used as a binder is known. For example, Patent Literatures 1 to 4 and Non-Patent Literature 1 describe nuclear particles in which a layer containing an active ingredient is arranged on the surface of the nuclear material by using the melt granulation method.

On the other hand, since the melt granulation method is greatly affected by the physical properties of the melt component, it is difficult to control the particle size of the granulated product. In addition, since the melt granulation method uses a melt component instead of a solvent, it is difficult to increase the content of the active ingredient in the granulated product, and as a result, the problems that the formulation inevitably becomes large and the medication adherence decreases arise.

### CITATION LIST

### PATENT LITERATURE

Patent Literature 1: Japanese laid-open patent publication No. 2017-1999
Patent Literature 2: Japanese laid-open patent publication No. 2015-199721
Patent Literature 3: Japanese laid-open patent publication No. 2015-71542
Patent Literature 4: Japanese laid-open patent publication No. H6-256169

### NON PATENT LITERATURE

Non Patent Literature 1: Chem. Pharm. Bull. 65, 726-731 (2017)

### SUMMARY OF INVENTION

### TECHNICAL PROBLEM

One of the problems of one embodiment of the present invention is to provide granules having a high content of an active ingredient and a high uniformity of particle size by using a melt granulation method. Another one of the problems of one embodiment of the present invention is to provide a preparation containing granules having a high content of an active ingredient and a high uniformity of particle size.

### SOLUTION TO PROBLEM

According to one embodiment of the present invention, a granule is provided that comprises a nuclear material, a melt component layer arranged on a surface of the nuclear material, and an active ingredient-containing layer arranged on a surface of the melt component layer, wherein the melt component layer contains a first melt component and the active ingredient-containing layer contains an active ingredient and a second melt component or a polymer having compatibility with the first melt component.

The second melt component may have a melting point of 100°C or lower and lower than a melting point of the first melt component.

The second melt component may have a melting point of 100°C or lower and higher than a melting point of the first melt component.

The polymer having compatibility may be selected from a group consisting of aminoalkyl methacrylate copolymers, ammonioalkyl methacrylate copolymers, methacrylic acid copolymers, hypromellose acetate succinates and polyvinylpyrrolidones when the first melt component is stearic acid or lauromacrogol.

The nuclear material may be spherical, and a particle size of the nuclear material may be larger than a particle size of the active ingredient, a particle size of the first melt component and the second melt component.

The nuclear material may have pores on a surface thereof, and the melt component layer may have a structure in which the first melt component is also arranged in the pores.

According to one embodiment of the present invention, a pharmaceutical preparation is provided that comprises any of the granules described above and one or more pharmaceutically acceptable additive agents.

The additive agent may be a disintegrant.

### ADVANTAGEOUS EFFECTS OF INVENTION

According to one embodiment of the present invention, granules having a high content of an active ingredient and a high uniformity of particle size are provided. Alternatively, according to one embodiment of the present invention, a preparation containing granules having a high content of an active ingredient and a high uniformity of particle size is provided.

### BRIEF DESCRIPTION OF DRAWINGS

Fig. 1 is a schematic diagram which shows a granule containing a nuclear particle according to one embodiment of the invention.
Fig. 2 is a schematic diagram which shows a granule containing a nuclear particle according to one embodiment of the invention.
Fig. 3 is a flow diagram which illustrates a producing method of a granule containing a nuclear particle according to one embodiment of the invention.
Fig. 4 is a flow diagram which illustrates a producing method of a granule containing a nuclear particle according to one embodiment of the invention.
Fig. 5(a) is a scanning electron microscope (SEM) image of granules of Example 1, (b) is a SEM image of the granules of Example 2, and (c) is a SEM image of the granules of Example 3, (d) is a SEM image of the granules of Example 4, (e) is a SEM image of the granules of Comparative Example 1, and (e) is a SEM image of the granules of Comparative Example 2.

### DESCRIPTION OF EMBODIMENTS

Hereinafter, a granule according to the present invention and a preparation using the same will be described with reference to drawings. In addition, the granule of the present invention and the preparation using the same are not construed as being limited to the description contents of the embodiments and examples shown below. In the drawings referred to in the present embodiment and the examples described later, the same parts or parts having the same functions are designated by the same reference numerals, and the repeated description thereof will be omitted.

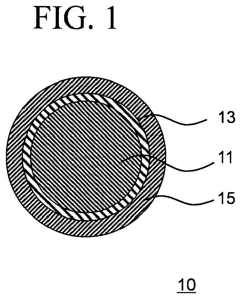

Fig. 1 is a schematic diagram (cross-sectional view) showing a granule 10 according to one embodiment of the present invention. The granule 10 contains a nuclear material 11, a melt component layer 13 arranged on the surface of the nuclear material 11, and an active ingredient-containing layer 15 arranged on the surface of the melt component layer 13.

The nuclear material 11 is a carrier for arranging the melt component layer 13 and the active ingredient-containing layer 15, and a substance which serves as a core for arranging the melt component layer 13 and the active ingredient-containing layer 15 when producing the granules 10. An adsorbent is used as the nuclear material 11 in order to obtain adhesion to the melt component layer 13. For examples, Amberlite IRP-64, ion exchange resin, kaolin, carmellose calcium, hydrated silicon dioxide, magnesium silicate, light anhydrous silicic acid, light liquid paraffin, silica soil, synthetic aluminum silicate, aluminum oxide, aluminum hydroxide, absorbent cotton, magnesium carbonate, precipitated calcium carbonate, dextrin, silicon dioxide, compound aluminum potassium silicate granules, bentonite, polyethylene fiber, magnesium aluminometasilicate, an adsorbent composed of medicated charcoal and the like can be use as the nuclear material 11.

The nuclear material 11 is preferably spherical in order to uniformly arrange the melt component layer 13 and the active ingredient-containing layer 15. Further, from the viewpoint of adhering the active ingredient, the particle size of the nuclear material 11 needs to be larger than the particle size of the active ingredient. The particle size of the nuclear material 11 is, for example, twice or more the particle size of the active ingredient, but is not limited thereto.

The melt component layer 13 is a layer arranged between the nuclear material 11 and the active ingredient-containing layer 15. The melt component layer 13 is a base layer for arranging the active ingredient-containing layer 15. In the above-mentioned patent document, a melt component and an active ingredient are directly arranged on a nuclear material, but when the active ingredient itself has weak adhesion to the nuclear material or the nuclear material has a low ability to support the active ingredient, it was inevitably necessary to add a large amount of melt component to the active ingredient, and it was not possible to obtain granules containing the active ingredient in a high content. On the other hand, in the present invention, by arranging the melt component layer 13 on the surface of the nuclear material 11 in the granule 10, more active ingredient can be attached to the melt component layer 13, and the content of the active ingredient in the granule 10 can be effectively increased.

As described above, the melt component (first melt component) constituting the melt component layer 13 is selected from oil-based additive agents. Since the melt component layer 13 is formed by a melt granulation method, the first melt component is selected from additive agents which are solid at room temperature. Considering the temperature range generally used in the melt granulation method, the first melt component is preferably selected from additive agents having a melting point of 100°C or lower, and preferably selected from additive agents which have a melting point in a temperature range in which denaturation of the active ingredient and significant increase of the related substances is not recognized. Examples of additive agents having such properties include, but are not limited to, glycerin monostearate, macrogol (polyethylene glycol), lauromacrogol, stearic acid, and the like. In addition, the first melt component is preferably selected from additive agents which do not denature the active ingredient and show a significant increase in related substances due to contact with the active ingredient.

The melt component layer 13 may be arranged on the surface of the nuclear material 11 in an amount in which the active ingredient-containing layer 15 can be arranged, and may be arranged on at least a part of the surface of the nuclear material 11. The melt component layer 13 preferably covers 90% or more of the surface of the nuclear material 11, and preferably covers the entire surface of the nuclear material 11. The thickness of the melt component layer 13 is not particularly limited, but it is preferable that the thickness of the melt component layer 13 is as thin as possible from the viewpoint of increasing the active ingredient content per granule 10. In one embodiment, it is preferable that the first melt component constituting the melt component layer 13 is also arranged in pores of the surface of the nuclear material 11. In one embodiment, the interface between the nuclear material 11 and the melt component layer 13 may have a structure in which the melt component constituting the melt component layer 13 enters from the surface of the nuclear material 11. In this case, the nuclear material 11 and the melt component layer 13 do not have to have a clear interface. By arranging the melt component not only on the surface of the nuclear material 11 but also in the pores connected to the surface of the nuclear material 11, the melt component layer 13 is imparted with an anchor effect on the nuclear material 11 and the adhesion of the melt component layer 13 on the nuclear material 11 is improved.

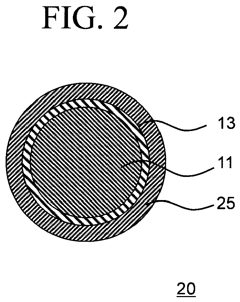

The active ingredient-containing layer is a layer containing the active ingredient and a second melt component or polymer, and is arranged on a surface of the melt component layer 13. Fig. 1 shows granules 10 in which the active ingredient-containing layer 15 contains the active ingredient and a second melt component. Fig. 2 shows granules 20 in which the active ingredient-containing layer 25 contains the active ingredient and a polymer having compatibility with the first melt component. In the granules 10 and 20, the active ingredient is not particularly limited. In methods for producing the granules 10 and 20, since a solvent, particularly water, is not used, a water-unstable active ingredient can be preferably used.

In the active ingredient-containing layer 15, the second melt component is an additive agent for binding the active ingredients to each other and binding the active ingredient to the surface of the melt component layer 13. Since the active ingredient-containing layer 15 is formed by the melt granulation method, the second melt component is selected from additive agents which are solid at room temperature. Considering the temperature range generally used in the melt granulation method, the second melt component is preferably selected from additive agents having a melting point of 100°C or lower, and preferably selected from additive agents which have a melting point in a temperature range in which denaturation of the active ingredient and significant increase of the related substances is not recognized. In the case where an additive agent having a melting point lower than a melting point of the first melt component is selected as the second melt component, when the active ingredient-containing layer 15 is formed by the melt granulation method, the active ingredient-containing layer 15 can be arranged on the surface of the melt component layer 13 without significantly affecting the surface structure of the melt component layer 13 or changing the surface structure of the melt component layer 13. On the other hand, in the case where an additive agent having a melting point higher than a melting point of the first melt component is selected as the second melt component, when the active ingredient-containing layer 15 is formed by the melt granulation method, the surface of the melt component layer 13 is slightly melted, and the interface between the melt component layer 13 and the active ingredient-containing layer 15 is fused, so that the adhesion of the active ingredient-containing layer 15 to the melt component layer 13 can be improved.

Examples of the additive agents used as the second melt component include stearic acid, glycerin monostearate, macrogol (polyethylene glycol), carnauba wax, hardened oil, lauromacrogol, palmitic acid, cetyl alcohol and the like, but are not limited thereto. The second melt component is preferably selected from additive agents which do not denature the active ingredient and show a significant increase in related substances due to contact with the active ingredient. From the viewpoint of adhering to the nuclear material 11, the particle size of the melt component needs to be smaller than the particle size of the nuclear material 11. Further, the melt component (second melt component) contained in the active ingredient-containing layer 15 may be the same additive agents as the melt component (first melt component) contained in the melt component layer 13, or may be different.

In one embodiment, in the granule 20, a polymer having compatibility with the first melt component can be used instead of the second melt component. The expression that the polymer is "compatible" with respect to the first melt component means that the first melt component and the polymer are not separated from each other. Alternatively, it indicates a state in which the polymer is dispersed in the first melt component, or a state in which the first melt component is dispersed in the polymer. In one embodiment, the state in which the melt component and the polymer are not separated can be confirmed by an increase in the viscosity of the mixture (liquid or semi-solid having fluidity) when the melt component and the polymer are mixed and the melt component is melted. By using a polymer having compatibility with the first melt component, the viscosity of the surface of the melt component layer 13 is further improved as compared with the case of using the second melt component, and the active ingredient-containing layer 25 can be adhered more stably. As a combination of a polymer having compatibility with the first melt component, when the first melt component is stearic acid or lauromacrogol, the polymer is an aminoalkyl methacrylate copolymer, an ammonioalkyl methacrylate copolymer, a methacrylic acid copolymer, a hypromellose acetate succinate or a polyvinylpyrrolidone can be preferably combined. More preferably, when the first melt component is stearic acid, an aminoalkyl methacrylate copolymer, an ammonioalkyl methacrylate copolymer or a polyvinylpyrrolidone can be combined as the polymer. Alternatively, when the first melt component is lauromacrogol, an aminoalkyl methacrylate copolymer, an ammonioalkyl methacrylate copolymer, a methacrylic acid copolymer or a hypromellose acetate succinate can be preferably combined.

When a polymer having compatibility with the first melt component is used instead of the second melt component, the content of the first melt component in the granules 20 is preferably equal to or higher than the content of the polymer. For example, in the granule 20, the blending ratio of the first melt component and the polymer is preferably 20 : 1 to 1 : 1 and more preferably 4 : 1 to 1 : 1.

The active ingredient-containing layer 15 and the active ingredient-containing layer 25 contain the active ingredient as a main component. The active ingredient-containing layer 25 preferably contains 50% by mass or more of the active ingredient with respect to the total mass of the active ingredient and the second melt component or polymer. In other words, in the active ingredient-containing layer 15 and the active ingredient-containing layer 25, it is preferable to contain a small amount of the second melt component or polymer on the surface of the melt component layer 13 in the extent which the active ingredient-containing layer 15 or the active ingredient-containing layer 25 can be formed. Thereby, the content of the active ingredient in the granules 10 and 20 can be effectively increased.

### [Method for producing granules 10]

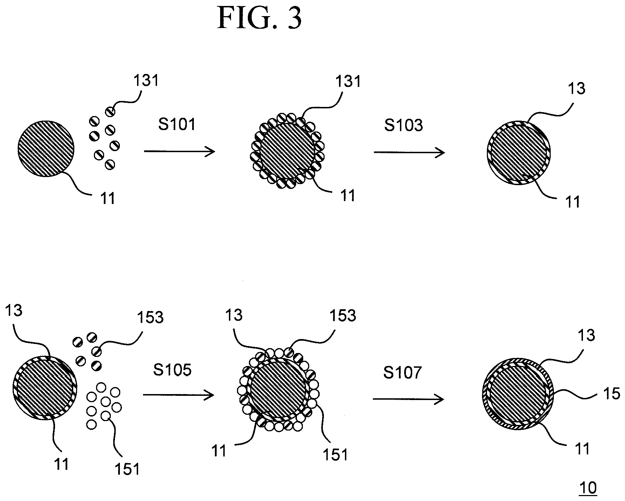

Fig. 3 is a flow diagram illustrating a method for producing granules containing nuclear particles according to one embodiment of the present invention. A nuclear material 11 and a first melt component 131 are mixed (S101), and the first melt component 131 is arranged on the surface of the nuclear material 11. Further, the first melt component 131 is melted by the melt granulation method to form the melt component layer 13 on the surface of the nuclear material 11 (S103). At this time, the nuclear material 11 and the first melt component 131 are heated to a temperature equal to or higher than the melting point of the first melt component 131. Considering the temperature range generally used in the melt granulation method, the heating temperature is 100°C or lower. Further, it is preferable that the first melt component 131 is arranged not only on the surface of the nuclear material 11 but also in the pores connected to the surface of the nuclear material 11, thereby imparting an anchor effect to the nuclear material 11 to the melt component layer 13 to improve the adhesion of the melt component layer 13 to the nuclear material 11.

The nuclear material 11 on which the melt component layer 13 is arranged is mixed with an active ingredient 151 and a second melt component 153 (S105), and the active ingredient 151 and the second melt component 153 are arranged on the surface of the melt component layer 13. Further, the second melt component 153 is melted by a melt granulation method to form an active ingredient-containing layer 15 on the surface of the melt component layer 13 (S107). At this time, the nuclear material 11 on which the melt component layer 13 is arranged, the active ingredient 151, and the second melt component 153 are heated to a temperature equal to or higher than the melting point of the second melt component 153. Considering the temperature range generally used in the melt granulation method, the heating temperature is 100°C or lower.

In one embodiment, in the case where an additive agent having a melting point lower than the melting point of the first melt component 131 is selected as the second melt component 153, when the active ingredient-containing layer 15 is formed by the melt granulation method, the active ingredient-containing layer 15 is formed by heating to a temperature higher than the melting point of the second melt component 153 and lower than the melting point of the first melt component 131, the active ingredient-containing layer 15 can be formed on the surface of the melt component layer 13 without significant effect on the surface structure of the melt component layer 13 or changing the surface structure of the melt component layer 13. On the other hand, in the case where an additive agent having a melting point higher than the melting point of the first melt component 131 is selected as the second melt component 153, when the active ingredient containing layer 15 is formed by the melt granulation method by heating to a temperature higher than the melting point of the second melt component 153, the surface of the melt component layer 13 is slightly melted, and the interface between the melt component layer 13 and the active ingredient-containing layer 15 is fused, then the adhesiveness of the active ingredient-containing layer 15 with respect to the melt component layer 13 can be improved. It is preferable to perform melt granulation in a temperature range in which the active ingredient 151 is not denatured and a significant increase in related substances is not observed.

### [Method for producing granules 20]

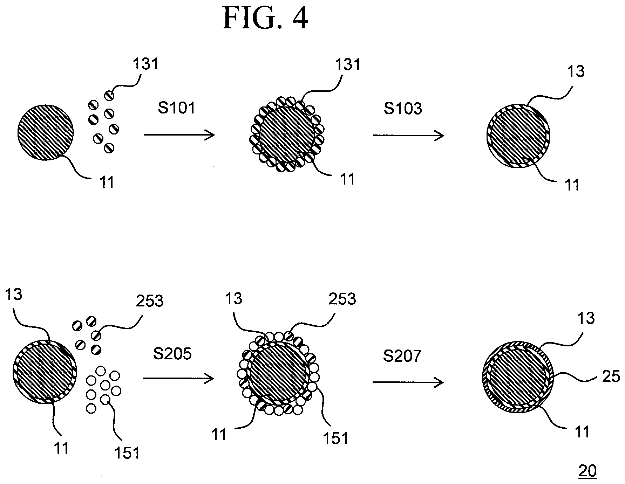

As described above, a polymer having compatibility with the first melt component can be used instead of the second melt component 153. Fig. 4 is a flow diagram illustrating a method for producing granules 20 containing nuclear particles according to one embodiment of the present invention. Since the producing method is the same as the producing method of the granules 10 described above until the melt component layer 13 is formed on the surface of the nuclear material 11 (S103), a detailed description thereof will be omitted.

The nuclear material 11 on which the melt component layer 13 is arranged is mixed with the active ingredient 151 and the polymer 253 having compatibility with the first melt component (S205), and the active ingredient 151 and the polymer 253 are arranged on the surface of the melt component layer 13. Further, the first melt component 131 is melted by a melt granulation method to form an active ingredient-containing layer 25 in which the active ingredient 151 and the polymer 253 are dispersed in the first melt component 131 on the surface of the melt component layer 13 (S207). At this time, the nuclear material 11 on which the melt component layer 13 is arranged, the active ingredient 151, and the polymer 253 are heated to a temperature equal to or higher than the melting point of the first melt component 131. Considering the temperature range generally used in the melt granulation method, the heating temperature is 100°C or lower.

In the present embodiment, the first melt component 131 arranged on the surface layer of the melt component layer 13 is melted, and the active ingredient 151 and the polymer 253 are dispersed in the surface layer of the melt component layer 13, so that the active ingredient-containing layer 25 is formed. In the present embodiment, since the polymer 253 is compatible with the first melt component, the active ingredient 151 and the polymer 253 do not separate from the first melt component 131 to form the active ingredient-containing layer 25. In the present embodiment, by using a polymer having compatibility with the first melt component, the viscosity of the surface of the melt component layer 13 is further improved as compared with the case of using the second melt component, and the active ingredient-containing layer 25 can be attached more stably.

### [Pharmaceutical preparation]

A pharmaceutical preparation using granule 10 or granule 20 can be produced. For example, granules 10 or 20 may be mixed with one or more known pharmaceutically acceptable additive agents to form a pharmaceutical composition. Alternatively, the pharmaceutical composition may be tableted into a tablet. Further, the pharmaceutical composition to which the disintegrant is added may be tableted to obtain an orally disintegrating tablet. Alternatively, the pharmaceutical composition may be encapsulated to form a capsule.

### EXAMPLE

### [Example 1]

Hydrated silicon dioxide (Fuji Silysia Chemical Ltd., Sylopure (registered trademark) P100) as a nuclear material and glycerin monostearate (RIKEN Vitamin Co., Ltd., RIKEMAL (registered trademark) S-100P) as the first melt component were used. 300 g of hydrated silicon dioxide and 480 g of glycerin monostearate were put into a high-speed stirring granulator (Fukae Kogyo Co., Ltd., High Speed Mixer, FS-GS-5J), and granulation was performed at an agitator rotation speed of 300 rpm, a chopper rotation speed of 1,500 rpm, and a water temperature of 75.0°C to 79.0°C for 11 minutes. At this time, the temperature of the additive agents was 69.5°C to 73.0°C.

195.0 g of obtained nuclear material on which the melt component layer was arranged on the surface, 372.2 g of sitagliptin phosphate as an active ingredient, and 27.0 g of stearic acid (NOF CORPORATION, Plant) as a second melt component were put into a high-speed stirring granulator (Fukae Kogyo Co., Ltd., high-speed mixer, FS-GS-5J), and granulation was performed at an agitator rotation speed of 150 rpm to 300 rpm, a chopper rotation speed of 1,500 rpm, and a water temperature of 74.9°C to 75°C for 22 minutes. At this time, the temperature of the additive agents was 68.4°C to 70.3°C.

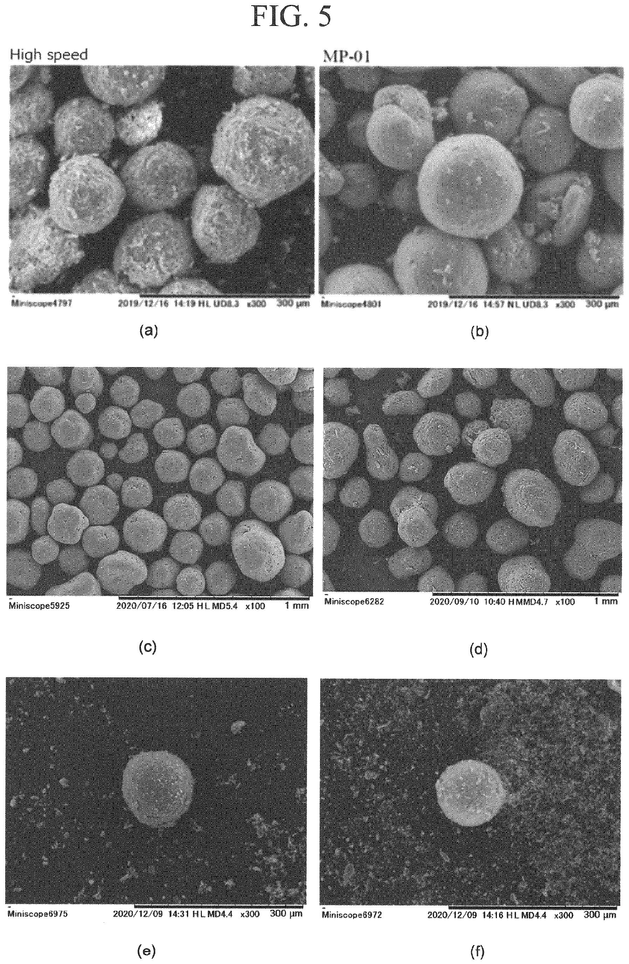

A scanning electron microscope (SEM) image of the obtained granules is shown in Fig. 5(a). In addition, the particle size of the granules was measured. The grain diameter measurement was performed using a laser diffraction / scattering method measuring device (Beckman Coulter Co., Ltd., LS 13 320). The measured grain diameter is shown in Table 1.

### [Example 2]

As Example 2, melt granulation was performed using a tumbling granulator using a nuclear material of Example 1 in which the melt component layer was arranged on the surface. 97.5 g of the nuclear material of Example 1 in which the melt component layer was arranged on the surface, 186.1 g of sitagliptin phosphate as the active ingredient, and 2.5 g of stearic acid (NOF CORPORATION., Plant) as the second melt component were put into a tumbling granulator (Powrex Corporation, MP-01), granulation was performed at a rotor rotation speed of 200 rpm to 500 rpm, an air supply air volume of 0.40 L/min to 0.55 L/min, and an air supply temperature of 89.5°Cto 90.9°C for 105 minutes. At this time, the temperature of the additive agents was 55.4°C to 65.7°C.

The SEM image of the obtained granules is shown in Fig. 5(b). The grain diameter of the granules of Example 2 is shown in Table 1.

**[Table 1]**

| Manufacturing equipment | Grain diameter (µm) | | |
|---|---|---|---|
| | D₁₀ | D₅₀ | D₉₀ |
| High-speed stirring granulator | 81 | 147 | 217 |
| Tumbling granulator | 114 | 156 | 215 |

The granules of Example 1 and Example 2 had an active ingredient content of about 60%, and it was clarified that a high content can be achieved. Further, from the results of Fig. 5(a) and Fig. 5(b), the granules of Example 1 and Example 2 are round particles and it became clear that the particle size of the granules is highly uniform due to the use of a nuclear material. With reference to Fig. 5(a), it was confirmed that the granules of Example 1 had irregularities on the surface. The granules of Example 1 having irregularities on the surface are expected to improve water conductivity. Further, referring to Fig. 5(b), since the surface of the granules of Example 2 is smooth, it is possible to consider applying a coating.

### [Examination of nuclear material]

In the production method of Example 1, the nuclear material was changed to form a melt component layer on the surface of the nuclear material. Fujisil (registered trademark) of Fuji Chemical Industries, Ltd. as hydrated silicon dioxide, Neusilin (registered trademark) US2 of Fuji Chemical Industries, Ltd. as magnesium aluminometasilicate, Celphere (registered trademark) CP102 of Asahi Kasei Corporation as microcrystalline cellulose, or NONPAREIL (registered trademark) 105 of FREUND CORPORATION as a mixture of lactose and microcrystalline cellulose was used. The examination results are shown in Table 2.

**[Table 2]**

| Nuclear material | First melt component | Production result |
|---|---|---|
| Hydrated silicon dioxide (Sylopure P100) | Glycerin monostearate (RIKEMAL S-100P) | ○ |
| Hydrated silicon dioxide (Fujisil) | | ○ |
| Magnesium aluminometasilicate (Neusilin US2) | | ○ |
| Microcrystalline cellulose (Celphere CP102) | | x |
| lactose / microcrystalline cellulose (NONPAREIL 105) | | x |

From the results in Table 2, when hydrated silicon dioxide and magnesium aluminometasilicate were used as the nuclear material, a melt component layer could be formed on the surface because the nuclear materials were an adsorbent with pores, but when other nuclear materials were used, it was difficult to form the melt component layer because it became a paste and the melt component layer could not be formed on the surface.

### [Examination of First Melt Component]

In the production method of Example 1, the first melt component was changed to form a melt component layer on the surface of the nuclear material. As the first melt component, macrogol 6000 (NOF Corporation, macrogol 6000 (P)), lauromacrogol (Nippon Surfactant Industry Co., Ltd.) or stearic acid (NOF Corporation, Plant) was used. The examination results are shown in Table 3.

**[Table 3]**

| Nuclear material | First melt component | Production result |
|---|---|---|
| Hydrated silicon dioxide (Sylopure P100) | Glycerin monostearate (RIKEMAL S-100P) | ○ |
| | Macrogol 6000 (P) | ○ |
| | Lauromacrogol | ○ |
| | Stearic acid (Plant) | ○ |

It was clarified that any of the oil-based additive agents which are solid at room temperature can form a melt component layer.

### [Examination of Second Melt Component]

In the production method of Example 1, the second melt component was changed to form an active ingredient-containing layer on the surface of the melt component layer. As the second melt component, stearic acid (NOF Corporation, plant), macrogol 6000 (NOF Corporation, macrogol 6000 (P)), or carnauba wax (Nippon Wax Co., Ltd., Polishing wax 105) was used. The examination results are shown in Table 4.

**[Table 4]**

| Nuclear material / melt component layer | Second melt component | Production result |
|---|---|---|
| Hydrated silicon dioxide (Sylopure P100) Glycerin monostearate (RIKEMAL S-100P) | Stearic acid | ○ |
| | Glycerin monostearate (RIKEMAL S-100P) | ○ |
| | Macrogol 6000 | ○ |
| | Carnauba wax | ○ |

It was clarified that any of the oil-based additive agents which are solid at room temperature can form an active ingredient-containing layer.

### [Examination of polymer to be used in place of the second melt component]

After mixing 1 g of the first melt component and 1 g of the polymer, the mixture was heated at 80°C for 2 hours. Further, 2 g of the first melt component was also heated at 80°C for 2 hours in the same manner, and the comparison of the viscosities of only the melt component and the melt component in which the polymer was mixed was evaluated by touch. Lauromacrogol (Nippon Surfactant Industry Co., Ltd.), stearic acid (NOF Corporation) or hardened oil (FREUND CORPORATION, Lubriwax) was used as the melt component. As polymers, aminoalkyl methacrylate copolymer E (Evonik, Eudragit (registered trademark) EPO), ammonioalkyl methacrylate copolymer RL (Evonik, Eudragit (registered trademark) RLPO), methacrylic acid copolymer L (Evonik, Eudragit (registered trademark)) L100-55), hypromellose acetate succinate (Shin-Etsu Chemical Co., Ltd., Shin-Etsu AQOAT (registered trademark) HPMC AS LF), or polyvinylpyrrolidone (BASF, K30) was used.

The evaluation results are shown in Table 1.

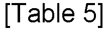

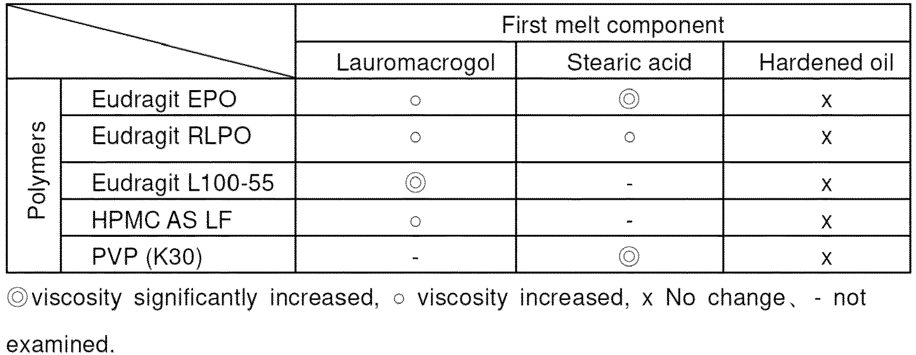

It was revealed that the aminoalkyl methacrylate copolymer, the ammonioalkyl methacrylate copolymer, the methacrylic acid copolymer, or the hypromellose acetate succinate increased in viscosity and showed compatibility when lauromacrogol was used as the melt component. In particular, it was revealed that the methacrylic acid copolymer showed excellent compatibility with lauromacrogol. Further, it was revealed that the aminoalkyl methacrylate copolymer, the ammonioalkyl methacrylate copolymer, or the polyvinylpyrrolidone increased in viscosity and showed the compatibility when stearic acid was used as a melt component. In particular, it was revealed that the aminoalkyl methacrylate copolymer and polyvinylpyrrolidone showed excellent compatibility with stearic acid. On the other hand, none of the polymers showed compatibility when hardened oil was used as the melt component.

### [Example 3]

It was examined whether granules could be produced by using a polymer having compatibility with the first melt component instead of the second melt component. 500.0 g of hydrated silicon dioxide (Fuji Silysia Chemical Ltd., Sylopure (registered trademark) P100) was used as the nuclear material, and 750.0 g of stearic acid (NOF CORPORATION, Plant) was used as the first melt component. Hydrous silicon dioxide and stearic acid were put into a high-speed stirring granulator (Fukae Kogyo Co., Ltd., High Speed Mixer, FS-GS-5J), and granulation was performed at an agitator rotation speed of 300 rpm, a chopper rotation speed of 500 rpm, and a water temperature of 78.4°C to 82.6°C for 17 minutes.

160.0 g of the obtained nuclear material on which the melt component layer was arranged on the surface, 496.4 g of sitagliptin phosphate as an active ingredient, and 48.0 g of aminoalkyl methacrylate copolymer E (evonic, Eudragit EPO) as a polymer having good compatibility with the first melt component were put into a tumbling fluid bed granulator (Powrex Corporation, MP-01), and granulation was performed at a rotor rotation speed of 400 rpm and an air supply temperature of 85°C for 25 minutes. At this time, the temperature of the additive agents was 62°C.

A scanning electron microscope (SEM) image of the obtained granules is shown in Fig. 5(c). In addition, the grain diameter of the granules was measured. The grain diameter measurement was performed using a laser diffraction / scattering method measuring device (Beckman Coulter Co., Ltd., LS 13 320). The measured grain diameters were D₁₀ = 165.0 µm, D₅₀ = 207.0 µm, and D₉₀ = 276.0 µm.

The granules of Example 3 had an active ingredient content of about 60%, and it was revealed that a high content of the active ingredient can be realized.

### [Example 4]

It was examined whether granules could be produced by using fexofenadine hydrochloride as the active ingredient instead of sitagliptin phosphate. 120.0 g of a nuclear material of Example 3 in which the melt component layer arranged on the surface, 240.0 g of fexofenadine hydrochloride as an active ingredient, and 40.0 g of aminoalkyl methacrylate copolymer E (evonic, Eudragit EPO) as a polymer having good compatibility with the first melt component were put into a tumbling fluid bed granulator (Powrex Corporation, MP-01), granulation was performed at a rotor rotation speed of 400 rpm and an air supply temperature of 80°C for 45 minutes. At this time, the temperature of the additive agents was about 60°C.

A scanning electron microscope (SEM) image of the obtained granules is shown in Fig. 5(d). In addition, the grain diameter of the granules was measured. The grain diameter measurement was performed using a laser diffraction / scattering method measuring device (Beckman Coulter Co., Ltd., LS 13 320). The measured grain diameters were D₁₀ = 169.4 µm, D₅₀ = 215.3 µm, and D₉₀ = 320.6 µm.

### [Comparative Example 1]

In order to examine whether the second melt component is essential for the production of high drug content granules, melt granulation was performed without using the second melt component. 436 g of hydrated silicon dioxide (Fuji Silysia Chemical Ltd., Sylopure (registered trademark) P100) was used as the nuclear material, and 654 g of stearic acid (NOF CORPORATION, Plant) as the first melt component were used. Hydrous silicon dioxide and stearic acid were put into a high-speed stirring granulator (Fukae Kogyo Co., Ltd., High Speed Mixer, FS-GS-5J), and granulation was performed at an agitator rotation speed of 300 rpm, a chopper rotation speed of 500 rpm, and a water temperature of 75.7°C to 78.5°C for 15 minutes. At this time, the temperature of the additive agents was 69.1°C to 71.9°C.

40.0 g of the obtained nuclear material on which the melt component layer was arranged on the surface and 124.1 g of sitagliptin phosphate as an active ingredient were put into a tumbling fluid bed granulator (Powrex Corporation, MP-01), granulation was performed at an air supply temperature of 85.0°C for 25 minutes. At this time, the temperature of the additive agents was 62°C.

A scanning electron microscope (SEM) image of the obtained granules is shown in Fig. 5(e). Since the melt component layer is arranged on the surface of the nuclear material, the active ingredient adheres slightly on the melt component layer, but since there is no second melt component, high drug content granules cannot be obtained.

### [Comparative Example 2]

In order to examine whether the first melt component is essential for the production of high drug content granules, melt granulation was performed without using the first melt component. Hydrous silicon dioxide (Fuji Silysia Chemical Ltd., Sylopure (registered trademark) P100) was used as the nuclear material, and aminoalkyl methacrylate copolymer E (Evonic, Eudragit EPO) was used as the second melting component. 16 g of hydrated silicon dioxide, 12 g of aminoalkyl methacrylate copolymer E and 124.1 g of sitagliptin phosphate were put into a tumbling fluid bed granulator (Powrex Corporation, MP-01), and granulation was performed at an air supply temperature of 85.0°C for 25 minutes. At this time, the temperature of the additive agents was 62°C

A scanning electron microscope (SEM) image of the obtained granules is shown in Fig. 5(f). Due to the absence of the first melt component, the active ingredient could not be arranged on the nuclear material at all.

### REFERENCES SIGNS LIST

10 granules, 11 nuclear material, 13 melt component layer, 15 active ingredient-containing layer, 20 granule, 25 active ingredient-containing layer, 131 first melt component, 151 active ingredient, 153 second melt component, 253 polymer

## Claims

1. A granule comprising:
a nuclear material; a melt component layer arranged on a surface of the nuclear material; and an active ingredient-containing layer arranged on a surface of the melt component layer,
wherein the melt component layer contains a first melt component,
the active ingredient-containing layer contains an active ingredient and a second melt component or a polymer having compatibility with the first melt component.

2. The granule according to claim 1, wherein the second melt component has a melting point of 100°C or lower and lower than a melting point of the first melt component.

3. The granule according to claim 1, wherein the second melt component has a melting point of 100°C or lower and higher than a melting point of the first melt component.

4. The granule according to claim 1, wherein
the polymer having compatibility is selected from a group consisting of aminoalkyl methacrylate copolymers, ammonioalkyl methacrylate copolymers, methacrylic acid copolymers, hypromellose acetate succinates and polyvinylpyrrolidones when the first melt component is stearic acid or lauromacrogol.

5. The granule according to claim 1, wherein the nuclear material is spherical, and
a particle size of the nuclear material is larger than a particle size of the active ingredient and a particle size of the second melt component.

6. The granule according to claim 1, wherein the nuclear material has pores on the surface thereof, and
the melt component layer has a structure in which the first melt component is also arranged in the pores.

7. A preparation comprising:
the granule according to any one of claims 1 to 6 and
one or more pharmaceutically acceptable additive agents.

8. The preparation according to claim 7, wherein the additive agent is a disintegrant.
